# FASCICULE DE BREVET EUROPEEN

(11) **EP 2 954 837 B1**
(45) Date de publication et mention de la délivrance du brevet: **03.05.2017**
(21) Numéro de dépôt: 15171950.7
(22) Date de dépôt: 12.06.2015
(51) Int. Cl.: A61B 3/14, A61B 5/117, G06K 9/00

(54) **DISPOSITIF D'ACQUISITION OPTIQUE POUR SYSTÈMES BIOMÉTRIQUES**
OPTISCHE ERFASSUNGSEINHEIT FÜR BIOMETRISCHE SYSTEME
OPTICAL ACQUISITION DEVICE FOR BIOMETRIC SYSTEMS

(30) Priorité: 13.06.2014 FR 1455447
(43) Date de publication de la demande: 16.12.2015
(73) Titulaire: MORPHO, 92130 Issy-Les-Moulineaux (FR)
(72) Inventeur: Rieul, François, 92130 Issy les Moulineaux (FR); PICARD, Sylvaine, 92130 Issy les Moulineaux (FR)
(74) Mandataire: Regimbeau

(56) Documents cités:
- EP-A1- 1 978 394
- WO-A1-2008/131201
- US-A1- 2010 290 668
- US-A1- 2011 032 481

## Description

### DOMAINE TECHNIQUE GÉNÉRAL ET ART ANTÉRIEUR

La présente invention est relative à l'acquisition optique dans des systèmes biométriques.

Elle trouve en particulier avantageusement application dans les systèmes biométriques utilisant des acquisitions d'images en vol (« OTF » ou « On The Fly » selon la terminologie anglo-saxonne généralement utilisée), par exemple pour l'identification ou l'authentification par reconnaissance faciale, acquisition d'iris, acquisition d'empreintes ou d'images veineuses, etc.

A titre d'exemple, le document US2010/290668 décrit un dispositif d'acquisition d'images pour système d'identification biométrique comprenant deux appareils de prise de vue pour l'acquisition d'une image d'un visage et d'une image des iris des yeux dans ce visage.

On recherche classiquement, dans les systèmes biométriques, à connaitre la distance séparant l'objet de la caméra. Ceci permet :
- de déconvoluer avec le bon filtre de déconvolution, et donc de supprimer le flou ;
- d'activer des systèmes de mise au point,
- de connaitre la résolution à laquelle on observe l'objet.

La connaissance de cette distance est souvent obtenue par projection d'une mire et par triangulation entre cette dernière et les droites de vue intervenant dans la formation des images. Cette méthode est très efficace mais a l'inconvénient d'être assez encombrante et de perturber l'image résultante par la présence de la mire.

Elle nécessite une parallaxe minimum entre la mire et la caméra et par conséquent un certain recul.

Elle est en outre couteuse en termes d'optique et d'électronique supplémentaires.

A titre d'exemple, le document US2011/032481 présente un dispositif d'acquisition d'images pour prendre une photographie du fond de l'oeil d'un individu comportant deux sources de lumière supplémentaires utilisées pour la mesure de la distance au fond de l'oeil en fonction de la taille de spots lumineux obtenus à l'aide des deux sources de lumières sur un plan d'imagerie après réflexion au fond de l'oeil. Le document WO2008/131201 décrit un dispositif d'acquisition d'images pour un système d'imagerie pour reconnaissance biométrique comportant une unité de traitement adaptée pour déterminer la distance d'un objet à analyser à partir de deux images ou par mesure acoustique ou ultrason, ou en fonction de paramètres géométriques mesurés sur une image tels qu'une distance entre les yeux, ou un diamètre d'iris. Le document EP1978394 décrit un système de prise de vue avec introduction d'une aberration optique adapté pour déterminer la distance d'un objet à analyser en fonction par exemple d'images acquises par plusieurs caméras.

Il est par ailleurs déjà connu en optique d'utiliser sur des caméras des ouvertures codées permettant de déterminer la distance à l'objet observé.

On pourra par exemple à cet égard se référer aux articles suivants :
- Zhou & Nayar "Coded Aperture Pairs for Depth from Defocus", 2009-Extended version : International Journal of Computer Vision, May 2011, Volume 93, Issue 1, pp 53-72
- Levin et al. "Image and Depth from a Conventional Camera with a Coded Aperture" - ACM transactions on graphics, Vol. 26, no. 3, article 70, July 2007.

Les ouvertures codées ont toutefois l'inconvénient de fortement dégrader la qualité de l'image qu'elles produisent.

Elles sont par conséquent généralement considérées comme incompatibles avec l'acquisition d'images de forte précision.

### PRÉSENTATION GÉNÉRALE DE L'INVENTION

Un but de l'invention est de pallier les inconvénients des techniques utilisées à ce jour pour la détermination d'une distance à l'objet dans le cas de systèmes biométriques.

Plus précisément, l'invention propose une solution qui est peu coûteuse et peu encombrante.

À cet effet, l'invention propose d'utiliser des diaphragmes à ouverture codée dans les systèmes biométriques.

Par diaphragme à ouverture codée, on entend ici et dans tout le présent texte tout diaphragme à ouverture autre qu'un trou centré et circulaire, et en particulier, mais non limitativement, toute ouverture de diaphragme consistant en plusieurs trous.

Plus précisément, l'invention propose un dispositif d'acquisition d'images pour système biométrique, comportant un ensemble optique et au moins un imageur pour l'acquisition d'une image d'un objet à analyser présenté devant ledit ensemble à une distance non déterminée de celui-ci, caractérisé en ce qu'il comporte au moins un diaphragme à ouverture codée, l'ensemble optique étant adapté pour permettre l'acquisition par le ou les capteurs :
- d'une part, d'une image de l'objet apte à être exploitée pour en déduire les informations biométriques recherchées et
- d'autre part, d'une image de l'objet acquise à travers l'ouverture codée du diaphragme,
ledit dispositif comportant en outre une unité de traitement adaptée pour déterminer la distance de l'objet à analyser en fonction de l'image de l'objet acquise à travers ladite ouverture codée du diaphragme.

Notamment, l'ensemble optique est avantageusement adapté pour que l'image de l'objet acquise à travers l'ouverture codée servant à la détermination de la distance soit dans une ou plusieurs bande(s) de fréquence différente(s) de la ou des bande(s) de fréquence de travail utilisée(s) pour l'acquisition de l'image porteuse des informations biométriques recherchées.

On exploite ainsi différentes couleurs pour les images acquises par les imageurs, alors que les acquisitions d'images biométriques se font généralement en ne prenant en compte qu'une seule bande de fréquence.

Avantageusement, l'ensemble optique comporte deux filtres de couleurs, l'un transmettant dans la bande de fréquence de travail utilisée pour l'acquisition de l'image porteuse des informations biométriques recherchée, l'autre transmettant dans une autre bande de fréquence, l'un au moins de ces deux filtres portant un diaphragme à ouverture codée.

Dans un mode de réalisation possible, l'ensemble optique comporte au moins deux diaphragmes :
- un diaphragme utilisé pour l'acquisition de l'image porteuse des informations biométriques recherchées, ledit diaphragme D étant doté :
   ∘ d'une ouverture (O) transparente au moins
      ▪ aux longueurs d'onde de travail (λw),
      ▪ aux longueurs d'onde (λd) de détermination de la distance
   ∘ d'une surface semi opaque en ce qu'elle est :
      ▪ opaque aux longueurs d'onde de travail (λw), et
      ▪ transparente aux longueurs d'onde (λd) de détermination de la distance et
      lesdites longueurs d'onde de travail (λw) et de détermination de la distance (λd) étant des longueurs d'onde distinctes,
- un second diaphragme à ouverture codée utilisé pour l'acquisition des informations de détermination de la distance, ledit diaphragme étant doté :
   ∘ d'une surface semi opaque en ce qu'elle est :
      ▪ opaque aux longueurs d'onde (λd) de détermination de la distance, et
      ▪ transparentes aux longueurs d'onde de travail (λw), et
   ∘ d'une ouverture codée (OC) transparente
      ▪ aux longueurs d'onde de travail (λw) et
      ▪ aux longueurs d'onde (λd) de détermination de la distance.

Selon un mode de réalisation préférentiel l'ouverture (O) du diaphragme est une ouverture centrée et circulaire permettant une acquisition de l'image de l'objet de qualité dans les longueurs d'onde de travail (λw).

Selon un mode de réalisation alternatif l'ouverture (O) du diaphragme D est une ouverture codée distincte de l'ouverture codée (OC) du diaphragme DOC, l'image de l'objet étant reconstituée à partir des informations acquises en sortie des deux diaphragmes.

Dans tous les cas de réalisations, les ouvertures (O) et (OC) sont indifféremment transparentes ou opaques aux autres longueurs d'ondes (λqc), étant entendu que ces dernières sont distinctes des longueurs d'onde de travail (λw) et des longueurs d'onde (λd) de détermination de la distance.

En variante, l'ensemble optique comporte une lame séparatrice renvoyant vers deux imageurs distincts une image de l'objet dans des bandes de fréquences différentes,
- l'une étant une ou des bande(s) de fréquence de travail utilisée(s) pour l'acquisition de l'image porteuse des informations biométriques recherchée,
- l'autre étant une ou des bande(s) de fréquence différente(s), un diaphragme centré étant disposé devant l'imageur recevant l'image dans la bande de fréquence de travail, le diaphragme à ouverture codée étant disposé devant l'autre imageur.

### PRÉSENTATION DES FIGURES

D'autres caractéristiques et avantages de l'invention ressortiront encore de la description qui suit, laquelle est purement illustrative et non limitative, et doit être lue en regard des figures annexées sur lesquelles :
- la figure 1 représente schématiquement un système d'acquisition conforme à un mode de réalisation possible pour l'invention ;
- la figure 2 illustre un système d'acquisition conforme à un autre mode de réalisation également possible.

### DESCRIPTION D'UN OU PLUSIEURS MODES DE MISE EN OEUVRE ET DE RÉALISATION

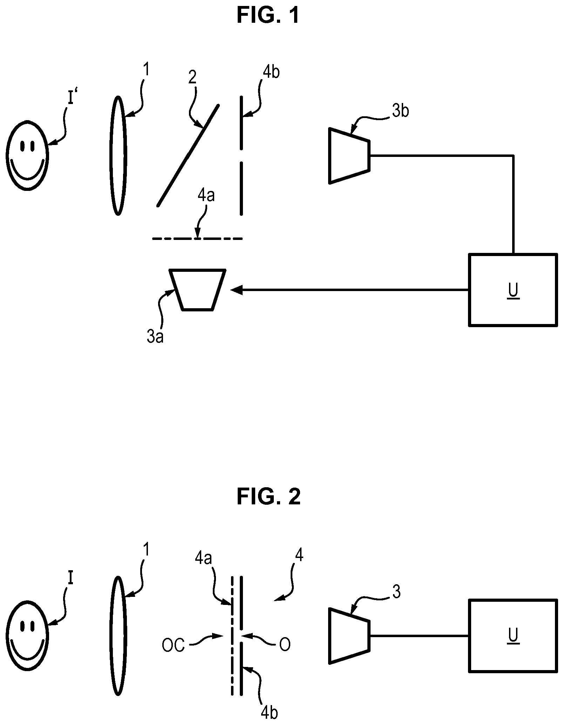

Dans l'exemple illustré sur la figure 1, le dispositif est destiné à acquérir une image du visage ou de l'iris de l'oeil d'un individu (objet I) passant devant ledit dispositif.

Bien entendu, il s'utiliserait de la même façon pour d'autres applications biométriques nécessitant des acquisitions « OTF » (acquisition « en vol » d'images de veines et ou d'empreintes, par exemple).

Le dispositif comporte une optique 1 et une lame séparatrice 2 et deux imageurs ou caméras 3a, 3b qui sont orientées à 90° l'une par rapport à l'autre et sur lesquelles sont renvoyées les images séparées par la lame 2.

La caméra 3a est associée à un diaphragme 4a à ouverture codée interposé entre ladite caméra et le la lame 2, tandis que la caméra 3b est associée à un diaphragme 4b classique centré circulaire, interposé entre ladite caméra 3b et la lame 2.

La lame 2 est par exemple une lame séparatrice qui filtre les couleurs, en transmettant sur la caméra 3a et la caméra 3b des images dans des bandes de fréquences différentes.

Les images transmises à la caméra 3b à diaphragme 4b classique sont des images dans une bande de fréquences de travail pour la biométrie considérée, c'est à dire une bande de fréquence qui permet l'exploitation des images acquises afin d'en déduire les informations biométriques recherchées.

Dans le cas d'acquisition d'images de veines ou d'images d'iris, il est classique de travailler dans le proche infra-rouge. La bande de fréquence de travail filtrée par la lame 2 et transmise sur la caméra 3b sera donc choisie pour être comprise entre [700 et 950 nm-]. Dans le cas d'acquisition d'images d'empreintes par contre, il est classique de travailler sur des images dans le domaine du bleu au vert. Pour ce type de biométrie, la bande de fréquence de travail filtrée par la lame 2 et transmise sur la caméra 3b sera donc choisie pour être comprise entre [450 à 650 nm].

Les images renvoyées à la caméra 3a à diaphragme à ouverture codé sont quant à elles des images dans une bande de fréquence autre et en particulier dans une bande de fréquence qui n'est pas utile pour l'obtention des informations biométriques.

Ainsi par exemple, cette bande de fréquence peut être dans l'IR et être comprise entre [800 à 950 nm-].

L'image ainsi reçue par la caméra 3a à ouverture codée est traitée pour déterminer la distance à laquelle l'objet (l'individu I) se trouve.

La caméra 3b reçoit quant à elle une image pleinement exploitable (sans perte d'information) pour l'analyse biométrique.

Le traitement des images est par exemple réalisé par une même unité de traitement U à laquelle sont les différentes images en sortie des caméras 3a, 3b sont transmises.

En variante, ainsi qu'illustré sur la figure 2, le système d'acquisition peut ne comporter qu'une seule caméra 3 multi-spectrale. Un ou plusieurs diaphragmes 4 sont interposés entre l'optique 1 et la caméra 3.

Cette solution a l'avantage d'être d'un coût moins important que la solution de la figure 1.

Notamment, dans un mode de réalisation possible, l'ensemble optique comporte à la fois un filtre 4b à diaphragme standard pour certaines couleurs correspondant à au moins bande de fréquences de travail pour la biométrie considérée et un filtre 4a diaphragme à ouverture codée pour d'autres couleurs permettant la détermination de la distance.

Les différentes images ainsi obtenues sont séparées entre les différents canaux couleurs de la caméra 3 et analysées par l'unité U qui :
- détermine la distance de l'objet I grâce aux images dans les couleurs correspondant au filtre 4a ;
- détermine les informations biométriques sur les images dans les couleurs du filtre 4b, en utilisant le cas échéant l'information sur la distance de l'objet obtenue grâce aux images du filtre 4a.

Les filtres 4a et 4b sont par exemple des filtres colorées (diapositives) avec des impressions de diaphragmes (diaphragme à ouverture codée dans le cas du filtre 4a ; diaphragme circulaire centré dans le cas du filtre 4b, par exemple).

Ces deux filtres 4a et 4b peuvent être réalisés en empilement et se présenter sous la forme d'un seul élément 4.

Dans un mode de réalisation préféré, les longueurs d'onde de travail λw et de détermination de la distance λd étant des longueurs d'onde distinctes, le filtre 4b qui constitue le diaphragme utilisé pour l'acquisition de l'image porteuse des informations biométriques recherchées comporte une ouverture O transparente au moins aux longueurs d'onde de travail λw et aux longueurs d'onde λd de détermination de la distance.

Cette ouverture O est ménagée dans une surface semi opaque qui est quant à elle opaque aux longueurs d'onde de travail λw, et transparente aux longueurs d'onde (λd) de détermination de la distance.

Le filtre 4a qui constitue le diaphragme à ouverture codée utilisé pour l'acquisition des informations de détermination de la distance comporte quant à lui une surface semi opaque qui est opaque aux longueurs d'onde λd de détermination de la distance, et transparente aux longueurs d'onde de travail (λw).

Il comporte en outre une ouverture codée OC transparente aux longueurs d'onde de travail λw et aux longueurs d'onde λd de détermination de la distance.

L'ouverture O du diaphragme 4b peut être une ouverture centrée et circulaire permettant une acquisition de qualité de l'image de l'objet dans les longueurs d'onde de travail λw.

Selon un autre mode de réalisation alternatif possible, l'ouverture O du diaphragme 4b est une ouverture codée distincte de l'ouverture codée OC du diaphragme 4a, l'image de l'objet étant reconstituée à partir des informations acquises en sortie des deux diaphragmes que constituent les filtres 4a, 4b.

Dans tous les cas de réalisations, les ouvertures O et OC sont indifféremment transparentes ou opaques aux autres longueurs d'ondes λqc qui ne sont utilisées ni pour la qualité de l'image, ni pour la détermination de distance. Ces longueurs λqc, qui sont distinctes des longueurs d'onde de travail λw et des longueurs d'onde λd utilisées pour la détermination de la distance, sont indifféremment filtrées ou non par les diaphragmes 4a, 4b.

De nombreux types d'ouvertures codées sont bien entendu envisageables.

On pourra par exemple utilisée des ouvertures codées du type de celles proposées dans l'article :
- Levin et al. "Image and Depth from a Conventional Camera with a Coded Aperture" - ACM transactions on graphics, Vol. 26, no. 3, article 70, July 2007.
- le calcul de distance mis en oeuvre par l'unité U étant alors du type de celui décrit dans ledit article.

Par ailleurs, pour améliorer encore la précision sur la distance, il est possible de projeter sur l'objet observé une image (mire / éclairage) à la longueur d'onde utilisée pour le filtre à ouverture codée, la projection se faisant dans l'axe de la caméra. Cette image permet d'ajouter des informations fréquentielles manquantes dans l'image utilisée pour la détermination de la distance.

Il est également possible de créer 2 ouvertures codées par des filtrages couleurs bien choisis. Dans ce cas on utilisera par exemple :
- Zhou & Nayar "Coded Aperture Pairs for Depth from Defocus", 2009-Extended version: International Journal of Computer Vision, May 2011, Volume 93, Issue 1, pp 53-72.

## Revendications

1. Dispositif d'acquisition d'images pour système biométrique, comportant un ensemble optique et au moins un imageur (3, 3a, 3b) pour l'acquisition d'une image d'un objet à analyser (I) présenté devant ledit ensemble à une distance non déterminée de celui-ci, ledit dispositif comportant au moins un diaphragme à ouverture codée (4a), l'ensemble optique étant adapté pour permettre l'acquisition par le ou les capteurs :
• d'une part, d'une image de l'objet apte à être exploitée pour en déduire les informations biométriques recherchées et
• d'autre part, d'une image de l'objet acquise à travers l'ouverture codée d'un diaphragme,
ledit dispositif comportant en outre une unité de traitement (U) adaptée pour déterminer la distance de l'objet à analyser en fonction de l'image de l'objet acquise à travers l'ouverture codée dudit diaphragme, **caractérisé en ce que** l'image de l'objet acquise à travers l'ouverture codée servant à la détermination de la distance est dans une ou plusieurs bande(s) de fréquence différente(s) de la ou des bande(s) de fréquence de travail utilisée(s) pour l'acquisition de l'image porteuse des informations biométriques recherchées, et **en ce que** l'ensemble optique comporte deux filtres de couleurs (4a, 4b), l'un transmettant dans la bande de fréquence de travail utilisée pour l'acquisition de l'image porteuse des informations biométriques recherchée, l'autre transmettant dans une autre bande de fréquence, l'un au moins de ces deux filtres (4a) portant un diaphragme à ouverture codée.

2. Dispositif selon la revendication 1, **caractérisé en ce que** les filtres sont réalisés en empilement.

3. Dispositif selon l'une des revendications 1 à 2, **caractérisé en ce qu'**il comporte un éclairage apte à projeter sur l'objet observé une image dans la bande de fréquence utilisée pour le diaphragme à ouverture codée.

4. Dispositif selon l'une des revendications 1 à 3, **caractérisé en ce que** la bande de fréquence du diaphragme à ouverture codée est dans le domaine du bleu.

5. Dispositif selon l'une des revendications 1 à 4, **caractérisé en ce que** l'ensemble optique comporte au moins deux diaphragmes :
- un diaphragme (4b) utilisé pour l'acquisition de l'image porteuse des informations biométriques recherchées, ledit diaphragme étant doté :
∘ d'une ouverture (O) transparente au moins
▪ aux longueurs d'onde de travail (λw),
▪ aux longueurs d'onde (λd) de détermination de la distance
∘ d'une surface semi opaque **en ce qu'**elle est :
▪ opaque aux longueurs d'onde de travail (λw), et
▪ transparente aux longueurs d'onde (λd) de détermination de la distance et
lesdites longueurs d'onde de travail (λw) et de détermination de la distance (λd) étant des longueurs d'onde distinctes,
- un second diaphragme à ouverture codée (4a) utilisé pour l'acquisition des informations de détermination de la distance, ledit diaphragme étant doté :
∘ d'une surface semi opaque **en ce qu'**elle est :
▪ opaque aux longueurs d'onde (λd) de détermination de la distance, et
▪ transparentes aux longueurs d'onde de travail (λw), et
∘ d'une ouverture codée (OC) transparente
▪ aux longueurs d'onde de travail (λw) et
▪ aux longueurs d'onde (λd) de détermination de la distance.

6. Dispositif selon la revendication 5, **caractérisé en ce que** l'ouverture (O) du diaphragme est une ouverture centrée et circulaire permettant une acquisition de l'image de l'objet de qualité dans les longueurs d'onde de travail (□w).

7. Dispositif selon l'une des revendications 5 à 6, **caractérisé en ce que** l'ouverture (O) du diaphragme est une ouverture codée distincte de l'ouverture codée (OC) du diaphragme, l'image de l'objet étant reconstituée à partir des informations acquises en sortie des deux diaphragmes.

8. Dispositif selon l'une des revendications 1 à 7, **caractérisé en ce que** l'ensemble optique comporte une lame séparatrice (2) renvoyant vers deux imageurs distincts (3a, 3b) une image de l'objet dans des bandes de fréquences différentes,
- l'une étant une ou des bande(s) de fréquence de travail utilisée(s) pour l'acquisition de l'image porteuse des informations biométriques recherchée,
- l'autre étant une ou des bande(s) de fréquence différente(s), un diaphragme centré étant disposé devant l'imageur recevant l'image dans la bande de fréquence de travail, le diaphragme à ouverture codée étant disposé devant l'autre imageur.

9. Utilisation d'un dispositif selon l'une des revendications précédentes pour l'acquisition d'images biométriques à la volée.

10. Utilisation d'un dispositif selon l'une des revendications précédentes pour l'identification ou l'authentification par reconnaissance faciale et/ou l'acquisition d'iris et/ou l'acquisition d'empreintes et/ou l'acquisition d'images veineuses.

## Patentansprüche

1. Bilderfassungsvorrichtung für biometrische Systeme, die eine optische Baugruppe und mindestens einen Bildaufnehmer (3, 3a, 3b) für die Erfassung eines Bildes eines zu untersuchenden Objekts (I) umfasst, das vor der Baugruppe in einem nicht bestimmten Abstand davon erscheint, wobei die Vorrichtung mindestens eine Blende mit codierter Apertur (4a) umfasst, wobei die optische Baugruppe angepasst ist, um die Erfassung von Folgendem durch den oder die Sensoren zu ermöglichen:
• einerseits eines Bildes des Objekts, das geeignet ist, ausgewertet zu werden, um davon die gesuchten biometrischen Informationen abzuleiten, und
• andererseits eines Bildes des Objekts, das durch die codierte Apertur einer Blende erfasst wurde,
wobei die Vorrichtung ferner eine Verarbeitungseinheit (U) umfasst, die angepasst ist, um den Abstand des zu analysierenden Objekts in Abhängigkeit von dem Bild des Objekts zu bestimmen, das durch die codierte Apertur der Blende erfasst wurde, **dadurch gekennzeichnet, dass** das Bild des durch die codierte Apertur erfassten Objekts, das zur Bestimmung des Abstands dient, sich in einem oder mehreren unterschiedlichen Frequenzbändern des oder der Arbeitsfrequenzbänder befindet, das oder die für die Erfassung des Bildes verwendet wurde/n, das die gesuchten biometrischen Informationen trägt,
und dadurch, dass die optische Baugruppe zwei Farbfilter (4a, 4b) umfasst, von denen das eine in dem Arbeitsfrequenzband durchlässt, das für die Erfassung des Bildes verwendet wird, das gesuchte biometrische Informationen trägt, das andere in einem anderen Frequenzband durchlässt, wobei mindestens eines dieser zwei Filter (4a) eine Blende mit codierter Apertur trägt.

2. Vorrichtung nach Anspruch 1, **dadurch gekennzeichnet, dass** die Filter in Stapelung ausgeführt sind.

3. Vorrichtung nach einem der Ansprüche 1 bis 2, **dadurch gekennzeichnet, dass** sie eine Beleuchtung umfasst, die geeignet ist, um auf das beobachtete Objekt ein Bild in dem für die Blende mit codierter Apertur verwendeten Frequenzband zu projizieren.

4. Vorrichtung nach einem der Ansprüche 1 bis 3, **dadurch gekennzeichnet, dass** das Frequenzband der Blende mit codierter Apertur sich im blauen Bereich befindet.

5. Vorrichtung nach einem der Ansprüche 1 bis 4, **dadurch gekennzeichnet, dass** die optische Baugruppe mindestens zwei Blenden umfasst:
- eine Blende (4b), die zur Erfassung des Bildes verwendet wird, das gesuchte biometrische Informationen trägt, wobei die Blende mit Folgendem versehen ist:
∘ einer Apertur (O), die für mindestens Folgendes durchlässig ist:
▪ gegenüber den Arbeitswellenlängen (λw),
▪ gegenüber den Wellenlängen (λd) zur Bestimmung des Abstands,
∘ einer halbundurchlässigen Fläche, dadurch, dass sie Folgendes ist:
▪ gegenüber den Arbeitswellenlängen (λw) undurchlässig, und
▪ gegenüber den Wellenlängen (λd) zur Bestimmung des Abstands durchlässig, und
wobei die Arbeitswellenlänge (λw) und die Wellenlänge zur Bestimmung des Abstands (λd) unterschiedliche Wellenlängen sind,
- eine zweite Blende (4a) mit codierter Apertur, die für die Erfassung der Informationen zur Bestimmung des Abstands verwendet wird, wobei die Blende mit Folgendem versehen ist:
∘ einer halbundurchlässigen Fläche, dadurch, dass sie Folgendes ist:
▪ gegenüber den Wellenlängen (λd) zur Bestimmung des Abstands undurchlässig, und
▪ gegenüber den Arbeitswellenlängen (λw) durchlässig, und
∘ einer codierten Apertur (OC), die gegenüber Folgendem durchlässig ist:
▪ gegenüber den Arbeitswellenlängen (λw) und
▪ gegenüber den Wellenlängen (λd) zur Bestimmung des Abstands.

6. Vorrichtung nach Anspruch 5, **dadurch gekennzeichnet, dass** die Apertur (O) der Blende eine zentrierte und kreisförmige Apertur ist, die eine Erfassung des Bildes des Objekts von Qualität in den Arbeitswellenlängen (λw) ermöglicht.

7. Vorrichtung nach einem der Ansprüche 5 bis 6, **dadurch gekennzeichnet, dass** die Apertur (O) der Blende eine codierte Apertur ist, die sich von der codierten Apertur (OC) der Blende unterscheidet, wobei das Bild des Objekts ausgehend von Informationen wiederhergestellt wird, die am Ausgang der zwei Blenden erfasst werden.

8. Vorrichtung nach einem der Ansprüche 1 bis 7, **dadurch gekennzeichnet, dass** die optische Baugruppe einen Strahlteiler (2) umfasst, der ein Bild des Objekts in unterschiedlichen Frequenzbändern in Richtung von zwei getrennten Bildaufnehmern (3a, 3b) zurücksendet,
- wobei das eine ein oder mehrere Arbeitsfrequenzbänder ist, das/die für die Erfassung des Bildes verwendet wird/werden, das die gesuchten biometrischen Informationen trägt,
- wobei das andere ein oder mehrere unterschiedliche Frequenzbänder ist, wobei eine zentrierte Blende vor dem Bildaufnehmer angeordnet ist, der das Bild in dem Arbeitsfrequenzband empfängt, wobei die Blende mit codierter Apertur vor dem anderen Bildaufnehmer angeordnet ist.

9. Verwendung einer Vorrichtung nach einem der vorhergehenden Ansprüche für die Erfassung von biometrischen On-the-Fly-Bildern.

10. Verwendung einer Vorrichtung nach einem der vorhergehenden Ansprüche zur Identifizierung oder Authentifizierung durch Gesichtserkennung und/oder Iris-Erfassung und/oder Erfassung von Abdrücken und/oder Erfassung von Venenbildern.

## Claims

1. A device for acquisition of images for a biometric system, comprising an optical assembly and at least one imager (3, 3a, 3b) for acquisition of an image of an object to be analysed (I) presented in front of said assembly at a non-determined distance from the latter, wherein said device comprises at least one diaphragm with coded aperture (4a), the optical assembly being adapted to allow acquisition by the sensor (s) :
• on one hand, of an image of the object capable of being exploited to deduce therefrom the searched biometric information, and
• on the other hand, of an image of the object acquired via the coded aperture of a diaphragm,
said device further comprising a processing unit (U) adapted to determine the distance of the object to be analysed as a function of the image of the object acquired via the coded aperture of said diaphragm, **characterized in that** the image of the object acquired via the coded aperture serving to determine the distance is acquired in one or more frequency bands separated from the work frequency band(s) used for acquisition of the image carrying the searched biometric information,
and **in that** the optical assembly comprises two colour filters (4a, 4b), one transmitting in the work frequency band used for acquisition of the image carrying the searched biometric information, the other transmitting in another frequency band, at least one of these two filters (4a) carrying a diaphragm with coded aperture.

2. The device according to claim 1, **characterized in that** the filters are made in a stack.

3. The device according to one of claims 1 to 2, **characterized in that** it comprises lighting capable of projecting onto the object observed an image in the frequency band used for the diaphragm with coded aperture.

4. The device according to one of claims 1 to 3, **characterized in that** the frequency band of the diaphragm with coded aperture is in the blue field.

5. The device according to one of claims 1 to 4, **characterized in that** the optical assembly comprises at least two diaphragms:
- one diaphragm (4b) used for acquisition of the image carrying the searched biometric information, said diaphragm being fitted with:
∘ an aperture (O) transparent at least
▪ to work wavelengths (λw),
▪ to wavelengths (λd) for determining the distance
∘ of a semi-opaque surface being:
▪ opaque to work wavelengths (λw), and
▪ transparent to wavelengths (λd) for determining the distance, and
said work wavelengths (λw) and wavelengths (λd) for determining the distance being separate wavelengths,
- a second diaphragm with coded aperture (4a) used for acquisition of the information for determining the distance, said diaphragm being fitted with:
∘ a semi-opaque surface:
▪ opaque to wavelengths (λd) for determining the distance, and
▪ transparent to work wavelengths (λw), and
∘ a coded aperture (OC) transparent
▪ to work wavelengths (λw), and
▪ to wavelengths (λd) for determining the distance.

6. The device according to claim 5, **characterized in that** the aperture (O) of the diaphragm is a centred and circular aperture enabling acquisition of an image of high quality of the object in the work wavelengths (λw).

7. The device according to one of claims 5 to 6, **characterized in that** the aperture (O) of the diaphragm is a coded aperture separate from the coded aperture (OC) of the diaphragm, the image of the object being reconstituted from information acquired at the output of the two diaphragms.

8. The device according to one of claims 1 to 7, **characterized in that** the optical assembly comprises a beam splitter (2) sending back to two separate imagers (3a, 3b) an image of the object in different frequency bands,
- one being one or more work frequency bands used for acquisition of the image carrying the searched biometric information,
- the other being one or more different frequency bands, a centred diaphragm being arranged in front of the imager receiving the image in the work frequency band, the diaphragm with coded aperture being arranged in front of the other imager.

9. Use of a device according to one of the preceding claims for acquisition of on-the-fly biometric images.

10. Use of a device according to one of the preceding claims for identification or authentication by facial recognition and/or iris acquisition and/or fingerprint acquisition and/or venous image acquisition.
